# EUROPEAN PATENT APPLICATION

(11) **EP 2 937 101 A1**
(43) Date of publication of application: **28.10.2015**
(21) Application number: 15159585.7
(22) Date of filing: 18.03.2015
(51) Int. Cl.: A61L 2/00

(54) **SEMI-FLUIDIC COMPOSITION FOR LUBRICATING, MOISTURE RETAINING, DISINFECTING, STERILIZING AND METHOD USING THE SAME**

(30) Priority: 19.03.2014 TW 103110388; 09.03.2015 TW 104107359
(71) Applicant: Pures Biotech Co., Ltd., Taichung City 40748 (TW)
(72) Inventor: Chang, Yi-Shou, 40748 Taichung City (TW)
(74) Representative: Baumann, Rüdiger Walter

(57) **Abstract**

A semi-fluid composition for lubricating, moisture retaining, disinfecting and sterilizing, which comprises of hypochlorous acid, thickening agent, emollient and water-binding agent, wherein the water and the thickening agent, emollient; and water-binding agent are mixed and stirred to form a hydrogel or a viscous substance, a method of using the semi-fluid composition for lubricating, moisture retaining, disinfecting and sterilizing, comprising following step: attaching the semi-fluid composition with effective amount to a subject-matter or applying the semi-fluid composition on the surface thereof, the subject is an adult intimacy product or a medical supply.

## Description

### FIELD OF THE INVENTION

The present invention relates to a composition and the method using the same, and particularly to a semi-fluid composition for lubricating, moisture retaining, disinfecting and sterilizing, and the method using the same.

### BACKGROUND OF THE INVENTION

Hypochlorous acid is an unstable weak acid, which could only exist in a solution. For example, it can dissolve in the water to form hypochlorous acid water. Hypochlorous acid is an extended-spectrum sterilizing ingredient which substantially exists in neutrophils of human body, and eliminates any kinds of protokaryon pathogen such as virus, bacteria, fungi. The leftover of hypochlorous acid after sterilization is water, which does not cause side effects like allergy or cancer etc. Hypochlorous acid was widely applied in our daily life as disinfectant and sterilizer, for example, the sterilizer of the endoscope in the hospital, the cleansing of vegetables and fruit, and the substitution fir iodine in wound treatment.

However, the hypochlorous acid solution has disadvantages such as its overactive character, rapidly decaying tendency, short action time, making it difficult to be applied to common use. In addition, the hypochlorous acid solution with high fluidity is hard to stay on its applied subject stably, which thus increases the risk of incomplete sterilized. Further, when applied to human body, affects and influences caused by hypochlorous acid other than disinfection and sterilization are issues widely noticed.

### SUMMARY OF THE INVENTION

Therefore, the object of the present invention is to break through the limit of traditional hypochlorous acid application, and provide a safe and durable, high-performance and extended-spectrum semi-fluid hypochlorous acid composition for disinfection and sterilization which is commercially applicable, with stable and slow release rate, moisture retaining, lubricating, protecting and repairing capabilities, and is suitable for extensive application.

In order to solve the problem in the prior art described above, the technical means adopted by the present invention provides a semi-fluid composition for lubricating, moisture retaining, disinfecting and sterilizing, which comprises water, hypochlorous acid, thickening agent, emollient, and water-binding agent. The water and the hypochlorous acid are mixed and stirred with the thickening agent, the emollient, and the water-binding agent to form a hydrogel or a viscous substance.

In one aspect of the present invention, the thickening agent is selected from a group consisting of agar powder, carboxyethyl cellulose (HEC), carboxymethyl cellulose, hydroxypropyl guar, polyacrylate, sodium alginate, xanthan gum, carbomer, and a mixture thereof.

In one aspect of the present invention, the emollient is selected from a group consisting of glyceryl caprylate, PEG-40 hydrogenated castor oil, sorbitan monolaurate, stearic acid, cetearyl alcohol, isopropyl myristate, triglycerides, myristic acid, palmitic acid, PEG-60 hydrogenated castor oil, glyceryl linoleate, cyclomethicone, dimethicone, hexyl laurate, isohexadecane, methyl glucose sesquioleate, and a mixture thereof.

In one aspect of the present invention, the water-binding agent is selected from a group consisting of glycerin, propylene glycol, butanediol, extract of hyaluronic acid, extract of aloe, vitamin B5, amino acid, ceramide, lecithin, trehalose, polysaccharide, sodium hyaluronate, mucopolysaccharides, sodium PCA, collagen, phospholipids, glycolsphingolipids, glycosaminoglycans, and a mixture thereof.

In one aspect of the present invention, it further comprises one or more nature plant oil, one or more preservatives, one or more essential oil, one or more essence, and a mixture thereof.

In one aspect of the present invention, the nature plant oil is selected from a group consisting of olive oil, grape seed oil, sunflower oil, avocado oil, sweet almond oil, shea butter, coconut oil, hawaii nut oil, and a mixture thereof.

In one aspect of the present invention, the preservative is selected from a group consisting of ethylhexylglycerin, tea tree essential oils, sorbic acid, and a mixture thereof.

In one aspect of the present invention, the water is micro-clustered water with hexagonal molecular clusters structure.

In one aspect of the present invention, a concentration of Hypochlorous acid in the water is in a range from 10 to 50 ppm by weight.

In one aspect of the present invention, a concentration of Hypochlorous acid in the water is in a range from 1 to 30 ppm by weight.

In one aspect of the present invention, a concentration of Hypochlorous acid in the water is less than or equal to 100 ppm by weight.

In one aspect of the present invention, a concentration of Hypochlorous acid in the water exceeds 100 ppm by weight.

In one aspect of the present invention, it further comprises animal serum protein, intercellular substance, mucopolysaccharides, nutrient broth of stem cells, ceramide, and glutaraldehyde.

In one aspect of the present invention, it further comprises high polymer.

In one aspect of the present invention, the high polymer comprises silicone gel.

In one aspect of the present invention, it further comprises antibacterial agent.

In one aspect of the present invention, the antibacterial agent is nanometer silver ion or chitosan.

In one aspect of the present invention, it further comprises biologically acceptable carrier.

In one aspect of the present invention, the biologically acceptable carrier is liposome.

In order to solve the problem of the prior art described above, another technical means adopted by the present invention provides an method of using the semi-fluid composition to lubricate, retain moisture, disinfect and sterilize, comprising steps: attaching the semi-fluid composition of any one of claims 1 to 19 with effective amount to a subject or applying the semi-fluid composition with effective amount to the surface of a subject, wherein the subject is an adult intimacy products or a medical supply.

Through the technical means adopted by the present invention, the semi-fluid composition for lubricating, moisture retaining, disinfecting and sterilizing is mixed with a thickening agent, making the hypochlorous acid water becomes semi-fluid state substance with low flowability such as gel, lotion and cream. The semi-fluid state not only allows the semi-fluid composition of the present invention to attach to the subject easily, but also endues the hypochlorous acid with stable activity and better disinfecting (with object such as HIV virus) and sterilizing effect. Also, the use of the hypochlorous acid without drug toxicity as a main ingredient of a composition for disinfection and sterilization, which is not only adoptable for the mucosal tissues of an organism because of its faint acidity, but also with the property of unlimited applicability to different types of pathogen, makes the semi-fluid composition of the present invention having undifferentiated and extended-spectrum sterilizing capability. In addition, the semi-fluid composition of the present invention has the effect of lubricating and moisture retaining by mixing with the emollient and the water-binding agent. Preferably, as per the applied subject and purpose of use, other ingredients can be added to the semi-fluid composition For example, the additives can be natural plant oil, preservative, essential oil, silicone gel, high polymer, repair factors, essence or the like, by which the semi-fluid composition of the present invention is possessed with the effects of protection, repair, water proofing, moisturization, extending the expiration date, or stress relief. In addition, the semi-fluid composition of the present invention can utilize Liposome Encapsulation Technology (LET) to improve the decay problem of hypochlorous acid by stably and slowly releasing the effective components, by which the expiration date of the semi-fluid composition of the present invention can be extended, making the semi-fluid composition of the present invention more commercially competitive.

The specific embodiment of the present invention is further explained by the detailed description of the preferred embodiment with appended tables as follows.

### DETAIL DESCRIPTON OF PREFERRED EMBODIMENT

The embodiment of the present invention is explained hereinafter. The explanation is not intended to limit the embodiment of the present invention, but only illustrates one of preferred embodiments thereof.

Because the hypochlorous acid only exists in solution, hypochlorous acid and water are directly referred as hypochlorous acid water hereinafter but not described separately.

A semi-fluid composition of the present invention for lubricating, moisture retaining, disinfecting and sterilizing comprises water, hypochlorous acid, thickening agent, emollient, and water-binding agent. The water and the hypochlorous acid are mixed and stirred with thickening agent, emollient, and water-binding agent to form a hydrogel or a viscous substance.

Specifically, there will be a fine sterilizing effect with the concentration of hypochlorous acid in the water equal to 1 ppm (parts per million) by weight. Because the hypochlorous acid contains chlorine, there will be a significant smell of chlorine once the concentration of hypochlorous acid in water exceeds 100 ppm. Therefore, if the semi-fluid composition is applied to daily use, the concentration of hypochlorous acid in water should better be less than or 100ppm by weight to avoid the smell of chlorine. Preferably, the concentration of hypochlorous acid in water from 10 to 50 ppm by weight endues the hypochlorous acid water with disinfecting and sterilizing effect but without causing stimulations in the organism. And if the semi-fluid composition is for medical use, such as the sterilization of medical kits, the concentration of hypochlorous acid in the water can exceed 100 ppm by weight. In this embodiment, the concentration of hypochlorous acid in water is 25 ppm by weight, and the pH value is from 2.0 to 7.0. Preferably, in other embodiments, if the semi-fluid composition is applied to some particular conditions, such as being applied to a relatively sensitive private part of an organism, the concentration of hypochlorous acid in water should better be from 1 to 3 ppm by weight, so as to reduce the possibility of causing stimulations in the organism.

The viscosity of the hypochlorous acid water can be adjusted via the thickening agent to form a hydrocolloid substance, like gel, or a viscous substance, like lotion or cream. The thickening agent can be selected from a group consisting of agar powder, carboxyethyl cellulose, carboxymethyl cellulose, hydroxypropyl guar, polyacrylate, sodium alginate, xanthan gum, carbomer, and a mixture thereof.

The semi-fluid composition of the present invention for lubricating, moisture retaining, disinfecting and sterilizing provides soft, moist and smooth feelings via a emollient. The emollient can be selected from a group consisting of glyceryl caprylate, PEG-40 hydrogenated castor oil, sorbitan monolaurate, stearic acid, cetearyl alcohol, isopropyl myristate, palmitic acid, PEG-60 hydrogenated castor oil, glyceryl linoleate, cyclomethicone, dimethicone, hexyl laurate, isohexadecane, methyl glucose sesquioleate, and a mixture thereof.

The semi-fluid composition of the present invention for lubricating, moisture retaining, disinfecting and sterilizing can prevent the moisture from evaporating via great water molecular absorbing capability of the water-binding agent. The water-binding agent can be selected from a group consisting of glycerin, propylene glycol, butanediol, extract of hyaluronic acid, extract of aloe, vitamin B5, amino acid, ceramide, lecithin, trehalose, polysaccharide, sodium hyaluronate, mucopolysaccharides, sodium PCA, collagen, phospholipids, glycolsphingolipids, glycosaminoglycans, and a mixture thereof.

Preferably, the semi-fluid composition of the present invention for lubricating, moisture retaining, disinfecting and sterilizing further comprises one or more natural plant oil, preservative, essential oil, essence, and a mixture thereof.

The preservative can suppress the growth of bacteria and extend the expiration date. In this embodiment, the preservative is ethylhexylglycerin. Certainly, the present invention is not limited to this. In other embodiments, the preservative can be selected from a group consisting of tea tree essential oils, sorbic acid, vitamin E and a mixture thereof. The tea tree essential oils acting as preservatives possess an effect of suppressing bacterial growth without doing harm to human body. Sorbic acid is non-poisonous and capable of suppressing the activation of mould, yeast, and aerobic bacteria, and can prevent the growth and reproduction of deleterious microorganisms such as clostridium botulinum, staphylococcus, and salmonella. Vitamin E is a natural antioxidant with the effect of antisepsis.

The natural plant oil has a moisturizing effect, and can be selected from a group consisting of olive oil, grape seed oil, sunflower oil, avocado oil, sweet almond oil, shea butter, coconut oil, Macadamia nut oil, and a mixture thereof.

Preferably, the water used in the semi-fluid composition of the present invention for lubricating, moisture retaining, disinfecting and sterilizing is a micro-clustered water. The micro-clustered water is a hexagonal shaped clustered water, which not only can extend the expiration date of the present invention, but also can pass through the water channel between cells of human body, and enter the cells to activate tissues via a special structure of the water channel between cell membrane, with hexagonal structure, formed by the enclosure of six protein subunits, in which a central hole is with a diameter of merely 2nm.

Preferably, the semi-fluid composition of the present invention for lubricating, moisture retaining, disinfecting and sterilizing can achieve the effect of refreshing, whitening, relaxing, estrus induction by adding different kinds of essential oil. For example, eucalyptus, rosemary, basil, tea tree, benzoin, bergamot, clary sage, cypress, grapefruit, jasmine, juniper, lavender, lemon, lemongrass, geranium, ylang-ylang, neroli, palmarosa, patchouli, vetiver, rosewood, basil, benzoin, Atlantic cedar, Roman chamomile and clary sage, sweet orange, petitgrain, rose, marjoram, frankincense, or benzoin.

The semi-fluid composition of the present invention for lubricating, moisture retaining, disinfecting and sterilizing can selectively add an essence to intensify aroma, and preferably, the essence is edible essence to ensure safety.

In other embodiments, the semi-fluid composition can form a thin film rapidly by spraying via adding high polymer such as liquid silicone gel, which renders the semi-fluid composition more convenient to use. For example, spraying the semi-fluid composition with high polymer like liquid silicone gel on a wound of an organism to form a thin film which isolates the wound from the outside environment, or applying the semi-fluid composition with high polymer to adult intimacy products, such as a condom, to form a protecting film which has the disinfecting and sterilizing effect.

For extending the sterilizing effect duration of the semi-fluid composition for lubricating, moisture retaining, disinfecting and sterilizing, preferably, in other embodiments, the semi-fluid composition further includes antibacterial agent, such as sterilizing material like nanometer silver ion or chitosan. Alternatively, the semi-fluid composition of the present invention for lubricating, moisture retaining, disinfecting and sterilizing can utilize the Liposome Encapsulation Technology (LET) to encapsulate hypochlorous acid into liposome, by which the semi-fluid composition of the present invention can slowly and stably release the hypochlorous acid ingredient.

In addition, the semi-fluid composition can add animal serum protein, intercellular substance, mucopolysaccharides, nutrient broth of stem cells, ceramide and glutaraldehyde to achieve the effect of repairing a biological tissue. Preferably, the semi-fluid composition can also in the meantime add the high polymer described above like liquid silicone gel, by which the semi-fluid composition of the present invention can have the effect of protecting and repairing tissues in the same time.

The semi-fluid composition of the present invention for lubricating, moisture retaining, disinfecting and sterilizing not only can be attached to the subject for a long time, but also have the effect of disinfection, sterilization, lubrication and moisture retaining, and thus is adoptable for being applied to organs of an organism, such as skin, penis, vagina, urethra, anus, oral cavity, nasal cavity, and ear cavity, or being attached to products for organisms such as adult intimacy products or medical supplies for human body. Preferably, as per the applied subject and the purpose of use, the semi-fluid composition of the present invention for lubricating, moisture retaining, disinfecting and sterilizing can selectively add natural plant oil, preservative, essential oil, silicone gel, essence, or other ingredients of the like, so as to further have various effects.

The present invention further comprises a method of using the semi-fluid composition for lubrication, moisture retaining, disinfection and sterilization, including following steps: attaching the semi-fluid composition with effective amount to a subject-matter or the surface thereof, wherein the subject is selected from a group consisting of organs of living organisms, adult intimacy products and medical supplies. Specifically, the organs of living organism include human anus, penis, urethra, vagina, oral cavity, nasal cavity, and ear cavity, etc. The adult intimacy products include condoms and vibrators, etc. The medical supplies include invasive medical devices such as a nasogastric, a gastroscopy, a colposcopy, or a colonoscopy etc.

Table 1 shows the embodiments of the semi-fluid composition of the present invention for lubricating, moisture retaining, disinfecting and sterilizing via adding different combinations of various additives.

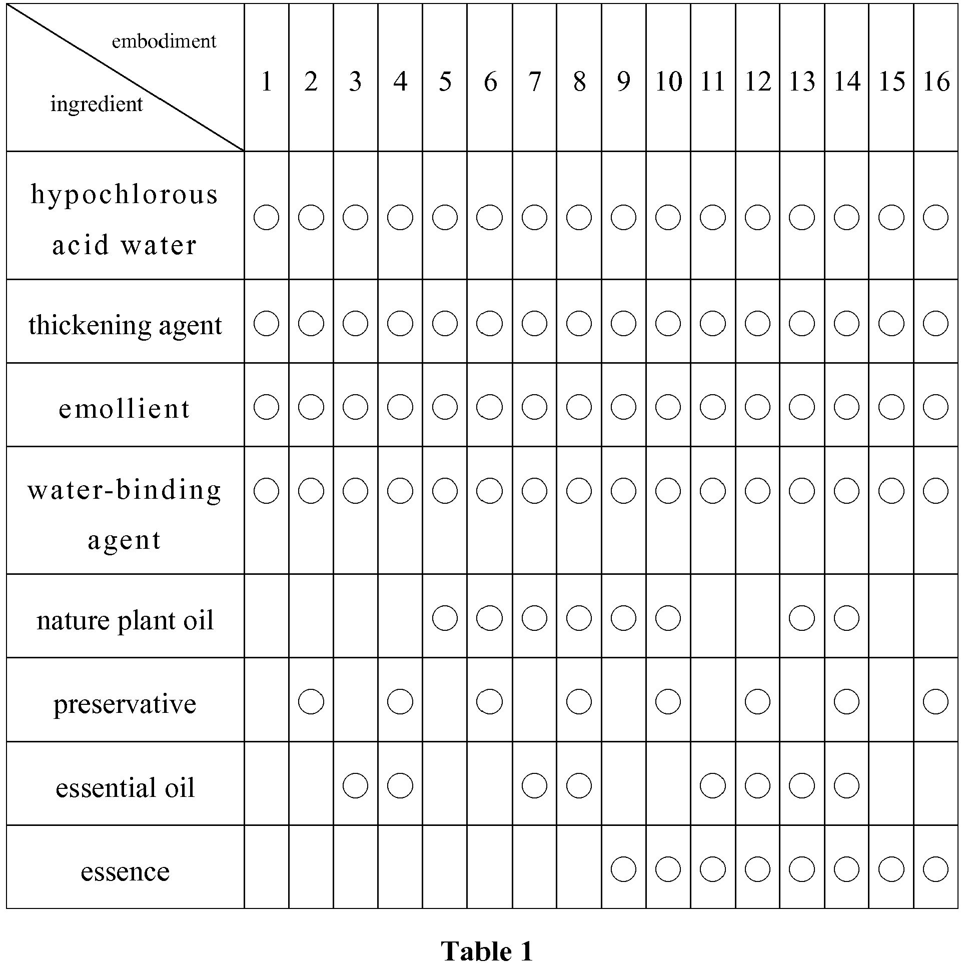

The semi-fluid composition of the present invention for lubricating, moisture retaining, disinfecting and sterilizing is used as a lubricant and a sterilizer for medical supplies such as a nasogastric, a gastroscopy, a colposcopy, a colonoscopy, or as a maintenance agent for female vagina to prevent itchy feelings caused from viral, bacterial or mould infection. The semi-fluid composition of the present invention for the above-mentioned purpose can be selected from embodiments 1 to 4 shown in table 1. The semi-fluid composition of the present invention for lubricating, moisture retaining, disinfecting and sterilizing used as a lubricant and a sterilizer for a nasogastric, a gastroscopy, a colposcopy, or a colonoscopy comprises 0.5~7wt% (percentage by mass) of the thickening agent, 0.5~20wt% of the emollient and 1~60wt% of the water-binding agent. The semi-fluid composition of the present invention for lubricating, moisture retaining, disinfecting and sterilizing used as a maintenance agent of femalevagina comprises 0.5~7wt% of the thickening agent, 1~17wt% of the emollient and 5~60wt% of the water-binding agent. Table 2 shows the specific ways of embodiment of embodiments 1 to 4. The semi-fluid composition of the present invention for lubricating, moisture retaining, disinfecting and sterilizing used as a lubricant for invasive medical devices or a maintenance agent for female vagina enters human body , therefore the thickening agent can be selected from cellulosic so as to decrease stimulations on the applied part. The cellulosic can be sodium alginate, hydroxypropyl guar, and sodium carboxymethyl as shown in embodiments 1 to 4 listed in table 2. The semi-fluid composition of the present invention for lubricating, moisture retaining, disinfecting and sterilizing can disinfect and sterilize the surface of medical supplies, and thus the composition is made semi-fluid so as to facilitate the process of entering the deep of anus, vagina and urethra. Besides, the lubrication and moisture retaining effect of the present invention can relieve the discomfort caused from the invasion of foreign object and avoid infections caused from an scrape of anus, vagina and urethra by the medical supplies when performing an medical examination. As shown in embodiments 2 to 4 listed in table 2, preferably, the semi-fluid composition of the present invention for lubricating, moisture retaining, disinfecting and sterilizing further comprises natural preservatives like tea tree essential oil, which extends the expiration date of the semi-fluid composition of the present invention for lubricating, moisture retaining, disinfecting and sterilizing. The percentage of the preservative in the semi-fluid composition of the present invention for lubricating, moisture retaining, disinfecting should be higher than 5wt% to possess a superior disinfection effect. In addition, as shown in embodiments 3 to 4 listed in table 2, the semi-fluid composition of the present invention for lubricating, moisture retaining, disinfecting and sterilizing can also include a lavender essential oil, wherein the percentage of the lavender essential oil preferably ranges from 0.5 to 1wt% to achieve the effect of calming and stress relief.

**Table 2**

| embodiment | 1 | 2 | 3 | 4 |
|---|---|---|---|---|
| | hypochlorous acid water | hypochlorous acid water | hypochlorous acid water | hypochlorous acid water |
| | 83% | 75% | 82.5% | 74.5% |
| thickening agent | sodium alginate | hydroxypropyl guar | sodium carboxymethyl cellulose | sodium carboxymethyl cellulose |
| | 1% | 2% | 1% | 1% |
| emollient | cyclic silicon ester | cyclic silicon ester | cyclic silicon ester | cyclic silicon ester |
| | 1% | 1% | 1% | 1% |
| water-binding agent | glycerin | glycerin | glycerin | glycerin |
| | 5% | 5% | 5% | 5% |
| water-binding agent | extract of hyaluronic acid | extract of hyaluronic acid | extract of hyaluronic acid | extract of hyaluronic acid |
| | 10% | 10% | 10% | 10% |
| | | tea tree essential oil (preservative) | lavender (essential oil) | tea tree essential oil (preservative) |
| | | 8% | 0.25% | 8% |
| | | | | lavender (essential oil) |
| | | | | 0.25% |

The semi-fluid composition of the present invention for lubricating, moisture retaining, disinfecting and sterilizing can be used as a lubricant for adult intimacy products like condoms, wherein the semi-fluid composition can be selected from embodiments 1 to 4, 11, 12, 15, or 16 in table 1. The semi-fluid composition of the present invention for lubricating, moisture retaining, disinfecting and sterilizing used as the lubricant for adult intimacy products like condoms comprises 0.5∼5wt% of the thickening agent, 0.5~20wt% of the emollient is and 0.5~60wt% of the water-binding agent. Table 3 shows specific ways of embodiment of embodiments 3, 4, 11, 12, 15 and 16. The semi-fluid composition of the present invention for lubricating, moisture retaining, disinfecting and sterilizing utilize the lubricant that possesses viscosity that can be attached to the surface of condoms for a long time, and the disinfecting and sterilizing effect of the semi-fluid composition of the present invention for lubricating, moisture retaining, disinfecting and sterilizing can prevent female vagina from viral, bacterial and mould infection. Moreover, the lubricating and moisture retaining effect of the semi-fluid composition of the present invention for lubricating, moisture retaining, disinfecting and sterilizing can relieve the dry and uncomfortable feelings of the female vagina caused from long time repeated friction in the process of sexual intercourse. Preferably, as shown in embodiments 4, 12 and 16 listed in table 3, the semi-fluid composition of the present invention for lubricating, moisture retaining, disinfecting and sterilizing can include preservatives to extend the expiration date of when being applied to adult intimacy products like condoms. The selected preservative can be sorbic acid, which is easy to be decomposed to carbon dioxide and water by human body and drained out of the body, which cause less harm to human body. In addition, as shown in embodiments 11, 12, 15, and 16 listed in table 3, the strawberry essence (or edible essence of other flavor) can be combined with the semi-fluid composition of the present invention for lubricating, moisture retaining, disinfecting and sterilizing to use, wherein the percentage of the edible essence is 1∼2wt%, such that the semi-fluid composition of the present invention for lubricating, moisture retaining, disinfecting and sterilizing can have various flavor like strawberry flavor. As shown in embodiments 3, 4, 11, and 12 listed in table 3, the semi-fluid composition of the present invention for lubricating, moisture retaining, disinfecting and sterilizing can include ylang-ylang(essential oil), and can be applied to adult intimacy products to achieve the effect of estrus induction. Certainly, the present invention is not limited to this, the semi-fluid composition of the present invention for lubricating, moisture retaining, disinfecting and sterilizing can be combined with the essential oil with different healing effect to present different effect. For example, the bergamot and lavender essential oil have the effect of stress relief. When being combined with the bergamot and lavender essential oil, the percentage of the essential oil should preferably be 0.25~3wt%. The semi-fluid composition of the present invention for lubricating, moisture retaining, disinfecting and sterilizing is applied to adult intimacy products like condoms, wherein the semi-fluid composition of the present invention not only provides the lubricating, moisture retaining, disinfecting and sterilizing effect, but also has more different effects via combining with various combinations of other additives.

**Table 3**

| embodiments | 3 | 4 | 11 | 12 | 15 | 16 |
|---|---|---|---|---|---|---|
| | hypochlorous acid water | hypochlorous acid water | hypochlorous acid water | hypochlorous acid water | hypochlorous acid water | hypochlo-r ous acid water |
| | 82.5% | 81.75% | 81.5% | 81% | 82% | 81.5% |
| thickening agent | carbomer | carbomer | carbomer | carbomer | carbomer | carbomer |
| | 1% | 1% | 1% | 1% | 1% | 1% |
| emollient | glyceryl caprylate | glyceryl caprylate | glyceryl caprylate | glyceryl caprylate | glyceryl caprylate | glyceryl caprylate |
| | 1% | 1% | 1% | 1% | 1% | 1% |
| water-binding agent | glycerin | glycerin | glycerin | glycerin | glycerin | glycerin |
| | 5% | 5% | 5% | 5% | 5% | 5% |
| water-binding agent | extract of hyaluronic acid | extract of hyaluronic acid | extract of hyaluronic acid | extract of hyaluronic acid | extract of hyaluronic acid | extract of hyaluronic acid |
| | 10% | 10% | 10% | 10% | 10% | 10% |
| | cananga odorata (essential oil) | sorbic acid (preservative) | cananga odorata (essential oil) | sorbic acid (preservative) | strawberry (edible essence) | sorbic acid (preservative) |
| | 0.5% | 0.5% | 0.5% | 0.5% | 1% | 0.5% |
| | | cananga odorata (essential oil) | strawberry (edible essence) | cananga odorata (essential oil) | | strawberry (edible essence) |
| | | 0.75% | 1% | 0.5% | | 1% |
| | | | | strawberry (edible essence) | | |
| | | | | 1% | | |

The semi-fluid composition of the present invention for lubricating, moisture retaining, disinfecting and sterilizing can be utilized as a skin care product like moisturizing lotion, which can be selected from embodiment 1 to 16 listed in table 1. The semi-fluid composition of the present invention for lubricating, moisture retaining, disinfecting and sterilizing used as a skin care product comprises 0.5∼10wt% of the thickening agent, 0.5∼15wt% of the emollient, and 2~60wt% of the water-binding agent. Preferably, If the semi-fluid composition of the present invention for lubricating, moisture retaining, disinfecting and sterilizing is used as a skin care product for long time use, the composition can be selected from embodiments 1, 3, 5, 7, 9, 11, 13 or 15 that do not add preservatives, so as to prevent harmful stimulations to the skin due to regular use of preservatives, because the hypochlorous acid water of the semi-fluid composition of the present invention for lubricating, moisture retaining, disinfecting and sterilizing already has disinfecting and sterilizing effects. Table 4 shows the specific ways of embodiment of embodiments 5, 7, 9, and 13 of the present invention without preservative. The utilization of the semi-fluid composition of the present invention for lubricating, moisture retaining, disinfecting and sterilizing as a skin care product can prevent the formation of acne on the skin and can also soften the skin and retain moisture thereof. If the applied skin is relatively dry, preferably, the semi-fluid composition of the present invention for lubricating, moisture retaining, disinfecting and sterilizing can include natural plant oil, wherein the percentage thereof is 5~30wt%. As shown in embodiments 5 and 7 listed in table 4, the semi-fluid composition of the present invention for lubricating, moisture retaining, disinfecting and sterilizing comprises shea butter (natural plant oil), which has small molecular size and is hard to be oxidized and can be easily absorbed by the skin. The oil can stay on the surface of the skin to form a protective layer, by which the oil has a stronger effect of moisturization.

**Table 4**

| embodiment | 5 | 7 | 9 | 13 |
|---|---|---|---|---|
| | hypochlorous acid water | hypochlorous acid water | hypochlorous acid water | hypochlorous acid water |
| | 59% | 59.25% | 71.75% | 72% |
| thickening agent | xanthan | xanthan | xanthan | xanthan |
| | 1% | 1% | 1% | 1% |
| thickening agent | hydroxypropyl guar | hydroxypropyl guar | hydroxypropyl guar | hydroxypropyl guar |
| | 1% | 1% | 1% | 1% |
| emollient | glyceryl caprylate | glyceryl caprylate | glyceryl caprylate | glyceryl caprylate |
| | 0.5% | 0.5% | 0.5% | 0.5% |
| emollient | PEG-40 hydrogenated castor oil | PEG-40 hydrogenated castor oil | PEG-40 hydrogenated castor oil | PEG-40 hydrogenated castor oil |
| | 0.5% | 0.25% | 0.75% | 0.5% |
| water-binding agent | glycerin | glycerin | glycerin | glycerin |
| | 3% | 3% | 3% | 3% |
| water-binding agent | propanediol | propanediol | propanediol | propanediol |
| | 5% | 5% | 5% | 5% |
| water-binding agent | butanediol | butanediol | butanediol | butanediol |
| | 4% | 4% | 4% | 4% |
| water-binding agent | extract of | extract of | extract of | extract of |
| | hyaluronic acid | hyaluronic acid | hyaluronic acid | hyaluronic acid |
| | 8% | 8% | 8% | 8% |
| water-binding agent | extract of aloe | extract of aloe | extract of aloe | extract of aloe |
| | 2% | 2% | 2% | 2% |
| water-binding agent | vitamin B5 | vitamin B5 | vitamin B5 | vitamin B5 |
| | 1% | 1% | 1% | 1% |
| | shea butter (natural plant oil) | shea butter (natural plant oil) | vanilla essence | rosemary (essential oil) 1% |
| | 15% | 14% | 2% | |
| | | rosemary (essential oil) | | vanilla essence |
| | | 1% | | 1% |

The semi-fluid composition of the present invention for lubricating, moisture retaining, disinfecting and sterilizing can also be applied to, besides from the areas mentioned above, a hand sanitizer, wherein the present invention can achieve the effect of not only disinfection and sterilization, but also softening and moisture retaining, which is different from the conventional hand sanitizers that comprise higher percentage of alcohol for sterilization, which brings dry and uncomfortable feelings.

The above description is only the explanation of the preferred embodiments of the present invention. A person with ordinary skills in the art can make various variations and modifications with reference to the above description. Nevertheless, the variations and modifications should fall into the scope of the present invention and the claims hereinafter.

## Claims

1. A semi-fluid composition for lubricating, moisture retaining, disinfecting and sterilizing, comprising:
water;
hypochlorous acid;
thickening agent;
emollient; and
water-binding agent,
wherein the water and the hypochlorous are mixed and stirred with the thickening agent, the emollient, and the water-binding agent to form a hydrogel or a viscous substance.

2. The semi-fluid composition of claim 1, wherein the thickening agent is selected from a group consisting of agar powder, carboxyethyl cellulose (HEC), carboxymethyl cellulose, hydroxypropyl guar, polyacrylate, sodium alginate, xanthan gum, carbomer, and a mixture thereof.

3. The semi-fluid composition of claim 1, wherein the emollient is selected from a group consisting of glyceryl caprylate, PEG-40 hydrogenated castor oil, sorbitan monolaurate, stearic acid, cetearyl alcohol, isopropyl myristate, palmitic acid, PEG-60 hydrogenated castor oil, glyceryl linoleate, cyclomethicone, dimethicone, hexyl laurate, isohexadecane, methyl glucose sesquioleate, and a mixture thereof.

4. The semi-fluid composition of claim 1, wherein the water binding agent is selected from a group consisting of glycerin, propylene glycol, butanediol, extract of hyaluronic acid, extract of aloe, vitamin B5, amino acid, ceramide, lecithin, trehalose, polysaccharide, polysaccharide, sodium hyaluronate, mucopolysaccharides, sodium PCA, collagen, phospholipids, glycolsphingolipids, glycosaminoglycans, and a mixture thereof.

5. The semi-fluid composition of claim 1, further comprising one or more natural plant oil, one or more preservative, one or more essential oil, one or more essence, or a mixture thereof.

6. The semi-fluid composition of claim 5, wherein the natural plant oil is selected from a group consisting of olive oil, grape seed oil, sunflower oil, avocado oil, sweet almond oil, shea butter, coconut oil, hawaii nut oil, and a mixture thereof.

7. The semi-fluid composition of claim 5, wherein the preservative is selected from a group consisting of ethylhexylglycerin, tea tree essential oils, sorbic acid, and a mixture thereof.

8. The semi-fluid composition of claim 1, wherein the water is micro-clustered water with hexagonal molecular clusters structure.

9. The semi-fluid composition of claim 1, wherein a concentration of hypochlorous acid in the water is in a range from 10 to 50 ppm by weight.

10. The semi-fluid composition of claim 1, wherein a concentration of hypochlorous acid in the water is in a range from 1 to 30 ppm by weight.

11. The semi-fluid composition of claim 1, wherein a concentration of hypochlorous acid in the water is less than or equal to 100 ppm by weight.

12. The semi-fluid composition of claim 1, wherein a concentration of hypochlorous acid in the water exceeds 100 ppm by weight.

13. The semi-fluid composition of claim 1, further comprising animal serum protein, intercellular substance, mucopolysaccharide, nutrient broth of stem cells, ceramide, and glutaraldehyde.

14. The semi-fluid composition of claim 1, further comprising high polymer.

15. The semi-fluid composition of claim 14, wherein the high polymer includes silicone gel.

16. The semi-fluid composition of claim 1, further comprising antibacterial agent.

17. The semi-fluid composition of claim 16, wherein the antibacterial agent is nanometer silver ion or chitosan.

18. The semi-fluid composition of claim 1, further comprising biologically acceptable carrier.

19. The semi-fluid composition of claim 18, wherein the biologically acceptable carrier is liposome.

20. A method of using a semi-fluid composition to lubricate, moisture retain, disinfect and sterilize, comprising steps:
attaching the semi-fluid composition of any one of claims 1 to 19 with effective amount to a subject or applying the semi-fluid composition with effective amount to the surface of the subject, wherein the subject is an adult intimacy product or a medical supply.
